# FASCICULE DE BREVET EUROPEEN

(11) **EP 3 858 256 B1**
(45) Date de publication et mention de la délivrance du brevet: **03.12.2025**
(21) Numéro de dépôt: 21163604.8
(22) Date de dépôt: 09.03.2017
(51) Int. Cl.: A61B 17/04, A61B 17/00

(54) **ANCRE À USAGE MÉDICAL DESTINÉE À ÊTRE INSÉRÉE DANS UNE PAROI OSSEUSE**
ANKER FÜR MEDIZINISCHE ZWECKE, DER ZUM EINSETZEN IN EINE KNOCHENWAND VORGESEHEN IST
ANCHOR FOR MEDICAL USE, DESIGNED TO BE INSERTED IN A BONE WALL

(30) Priorité: 10.03.2016 FR 1652026; 17.10.2016 FR 1660055
(43) Date de publication de la demande: 04.08.2021
(62) Demande divisionnaire de: 17711780.1
(73) Titulaire: Dedienne Sante, 34130 Mauguio (FR); Chaouat, Gilles, 77330 Ozoir La Ferrière (FR); Decrette, Edouard, 27930 Gauciel (FR); Cohen, Gilles, 75017 Paris (FR); Fischer, Marc, 92100 Boulogne Billancourt (FR); Zeitoun, Jean-Marc, 75006 Paris (FR)
(72) Inventeur: Chaouat, Gilles, 77330 Ozoir La Ferrière (FR); Decrette, Edouard, 27930 GAUCIEL (FR); Cohen, Gilles, 75017 Paris (FR); Fischer, Marc, 92100 Boulogne Billancourt (FR); Zeitoun, Jean-Marc, 75006 Paris (FR); Lefief, Damien, 30320 Poulx (FR)
(74) Mandataire: LLR

(56) Documents cités:
- EP-A1- 1 917 917
- FR-A1- 2 682 867
- US-A1- 2005 245 932
- US-A1- 2007 005 068
- US-A1- 2008 051 836
- US-A1- 2012 059 429

## Description

La présente invention concerne une ancre à usage médical, destinée à être insérée dans une paroi osseuse.

Cette ancre permet de fixer un ou plusieurs fils de suture à un os pour, notamment réinsérer un tendon ou un ligament sur un os ; elle est particulièrement destinée à permettre de réinsérer, sur un humérus, un ou plusieurs des tendons de la coiffe des rotateurs de l'articulation de l'épaule.

Une ancre à usage médical est connue de FR2682867 A1.

Il est bien connu, dans diverses indications, de refixer un tendon ou un ligament sur un os au moyen d'une ancre insérée dans la paroi de cet os, cette ancre étant reliée à un ou plusieurs fils de suture qui permettent de suturer le tendon ou ligament à l'os.

Pour relier le fil de suture à une ancre, il est connu de prévoir un passage transversal aménagé au travers de l'ancre, au travers duquel le fil de suture est engagé. Ce type d'assemblage a pour inconvénient de générer des détériorations au fil lors de la mise en place de l'ancre, dues aux frottements entre l'ancre et la paroi osseuse. Ces détériorations peuvent conduire à une rupture de ce fil.

Il est également connu de prévoir un anneau proximal sur une ancre, auquel le fil de suture est noué. Ce type d'ancre présente un risque notable de rupture du fil au niveau du nœud.

Pour remédier à cet inconvénient, il a été conçu des ancres dites *"knotless",* dans lesquelles le fil est retenu sur l'ancre par pincement ou coincement. Les ancres existantes n'excluent cependant pas tout risque de détérioration du fil lors de la mise en place de l'ancre, particulièrement lorsque cette dernière est vissée, ou des risques de sectionnement du fil au niveau de son point de liaison à l'ancre.

La présente invention vise à remédier à cet inconvénient essentiel.

Par ailleurs, un certain nombre des ancres existantes présente une résistance à l'arrachement relativement incertaine. Ce problème se pose en particulier pour des ancres destinées à la réinsertion des tendons de la coiffe des rotateurs d'une articulation de l'épaule, compte tenu de la tension notable exercée sur le ou les fils de suture, et donc sur la ou les ancres, par ces tendons lors du mouvement de l'articulation.

La présente invention vise également à remédier à cet inconvénient.

L'objectif principal de l'invention est donc de fournir une ancre à laquelle un ou plusieurs fils de suture peuvent être parfaitement fixés et retenus, avec un risque réduit de cisaillement ou de rupture de ce ou ces fils.

Un autre objectif de l'invention est de fournir une ancre apte à être parfaitement fixée à une paroi osseuse.

L'ancre concernée comprend, de manière connue en soi, deux pièces destinées à être assemblées l'une à l'autre, à savoir une pièce extérieure, destinée à être fixée à l'os, qui forme une cavité longitudinale interne à travers elle, et une pièce intérieure, destinée à être insérée et retenue dans ladite cavité de la pièce extérieure, qui forme un conduit transversal la traversant de part en part ; au moins un fil de suture est destiné à être engagé dans ladite cavité par le côté proximal de la pièce extérieure, à être engagé au travers dudit conduit transversal et à ressortir de ladite pièce extérieure, par ledit côté proximal.

Selon l'invention, qui est définie par la revendication 1,
- la pièce extérieure forme au moins une première surface de coincement disposée dans la direction longitudinale de la pièce extérieure et la pièce intérieure forme au moins une deuxième surface de coincement située du côté proximal par rapport au conduit transversal qu'elle comprend, cette deuxième surface de coincement étant disposée dans la direction longitudinale de la pièce intérieure ;
- ledit au moins un fil de suture est destiné à s'étendre le long de ladite première surface de coincement et le long de ladite deuxième surface de coincement ;
- la pièce intérieure est mobile par rapport à la pièce extérieure entre une position distale de non blocage du fil de suture, dans laquelle ladite deuxième surface de coincement est à une distance de ladite première surface de coincement ne réalisant aucun coincement de ce fil, et une position proximale de blocage du fil de suture, dans laquelle ladite deuxième surface de coincement est à une distance de ladite première surface de coincement réalisant un coincement de ce fil.

Il sera compris que les termes "proximal" et "distal" utilisés ci-dessus et dans l'ensemble de la présente description sont à considérer, de façon classique, par rapport au sens d'insertion de l'ancre dans une paroi osseuse, le terme "proximal" désignant un emplacement plus proche d'un praticien par rapport à ce sens d'insertion et "distal" désignant un emplacement plus éloigné de ce praticien par rapport à ce même sens d'insertion.

L'invention fournit une ancre dans laquelle les brins du ou des fils de suture se trouvent à l'intérieur de l'ancre lors de la mise en place de l'ancre et sont donc préservés de détériorations qui, autrement, seraient générées par les parois de l'ancre et de l'os lors de cette mise en place. La pièce extérieure peut alors comprendre extérieurement une structure permettant un ancrage renforcé à l'os ; il peut s'agir de nervures d'ancrage ayant une hauteur importante, et/ou d'une section transversale non circulaire de la pièce extérieure, notamment oblongue, faisant que la pièce extérieure est apte, dans une première position angulaire, à être engagée dans un trou de réception aménagé dans l'os, puis à être pivotée en force dans ce trou, jusqu'à une autre position angulaire, décalée de l'ordre de quatre-vingt-dix degrés par rapport à ladite première position angulaire, de façon à coincer l'ancre dans le trou.

Avant mise en place de l'ancre, la pièce intérieure peut ne pas être assemblée à la pièce extérieure, faisant que chaque fil de suture peut être facilement engagé sur l'ancre.

Une fois l'ancre mise en place sur l'os, une traction exercée sur les brins du ou des fils de suture permet de déplacer la pièce intérieure par rapport à la pièce extérieure vers ladite position proximale de blocage, réalisant un blocage de ce ou ces fils par coincement entre lesdites première et deuxième surfaces de coincement.

Ces surfaces de coincement, disposées longitudinalement, permettent d'exercer une pression sur une portion relativement importante du ou des fils, assurant un parfait coincement de ce ou ces fils, sans risque de cisaillement.

Selon l'invention, ladite pièce extérieure comprend deux dites premières surfaces de coincement, diamétralement opposées, et ladite pièce intérieure comprend deux dites deuxièmes surfaces de coincement correspondantes.

Ainsi les deux brins de chaque fil de suture sont coincés dans l'ancre par deux paires de surfaces de coincement, une située avant le passage du ou des fils au travers dudit conduit transversal, et l'autre située après ce passage.

Au moins une desdites première et deuxième surfaces de coincement est inclinée par rapport à l'axe longitudinal de l'ancre.

Le coincement du ou des fils de suture est ainsi réalisé par réduction progressive, lors du déplacement de ladite pièce intérieure vers ladite position proximale de blocage, de l'espace existant entre lesdites première et deuxième surface de coincement.

De préférence, ladite pièce extérieure forme au moins une première surface d'engagement, distincte de ladite première surface de coincement, et ladite pièce intérieure forme au moins une deuxième surface d'engagement, distincte de ladite deuxième surface de coincement, ces surfaces d'engagement respectives venant en engagement mutuel dans ladite position proximale de blocage et réalisant une immobilisation de ladite pièce intérieure par rapport à ladite pièce extérieure dans cette position.

Un risque de retour de ladite pièce intérieure dans ladite position distale de non blocage est ainsi éliminé.

De préférence, ces première et deuxième surfaces d'engagement sont inclinées par rapport à l'axe longitudinal de l'ancre de manière à être aptes à venir, lorsque la pièce intérieure est déplacée dans le sens proximal à l'intérieur de la pièce extérieure, dans un état de coincement mutuel qui détermine ladite position proximale de blocage, et lesdites surfaces de coincement respectives sont aménagées de telle sorte qu'il existe entre elles, dans cette position proximale de blocage, un espace dont l'épaisseur est de l'ordre de 10 à 40% du diamètre d'un fil de suture.

Les surfaces d'engagement sont ainsi aménagées de manière à ce que leur coincement mutuel intervienne avant que lesdites surfaces de coincement respectives réalisent un écrasement excessif du ou des fils de suture. Le risque d'un tel écrasement excessif est éliminé de cette façon.

Selon une forme de réalisation préférée de l'invention,
- ladite pièce extérieure et ladite pièce intérieure présentent des moyens respectifs de positionnement angulaire de ladite pièce intérieure dans ladite pièce extérieure, plaçant ledit conduit transversal de la pièce intérieure dans une position angulaire déterminée par rapport à la pièce extérieure ;
- ladite pièce extérieure présente une cavité conique femelle dans laquelle sont aménagés deux conduits diamétralement opposés, de passage des brins du ou des fils de suture destinés à être reliés à l'ancre ; ces conduits sont aménagés sur un diamètre qui est parallèle à l'axe dudit conduit transversal lorsque ladite pièce intérieure se trouve dans ladite position angulaire déterminée ; les parois délimitant ces conduits sur le côté radialement extérieur de la pièce extérieure forment lesdites premières surfaces de coincement ; les surfaces délimitant ladite cavité conique femelle entre ces conduits forment deux dites premières surfaces d'engagement ;
- ladite pièce intérieure présente une portion tronconique mâle de laquelle font saillie deux bossages diamétralement opposés ; ces bossages sont aménagés sur un diamètre parallèle à l'axe dudit conduit transversal ; ces bossages sont destinés à s'engager dans lesdits conduits lors du déplacement de ladite pièce intérieure de ladite position distale de non blocage à ladite position proximale de blocage, et à coincer les brins du ou des fils de suture entre eux et lesdites parois délimitant les conduits vers l'extérieur dans la direction radiale ; les bossages constituent lesdites deuxièmes surfaces de coincement, et les surfaces de ladite portion tronconique mâle situées en dehors de ces bossages forment deux dites deuxièmes surfaces d'engagement, destinées à venir en engagement avec lesdites premières surfaces d'engagement dans ladite position proximale de blocage.

Lesdits moyens respectifs de positionnement peuvent notamment être constitués par deux rainures longitudinales diamétralement opposées aménagées dans ladite pièce extérieure et par des nervures correspondantes aménagés sur la pièce intérieure, ces nervures formant des patins destinés à être reçus dans la glissière que forment les rainures.

Avantageusement, la pièce extérieure présente deux lumières longitudinales diamétralement opposées, situées en regard des ouvertures dudit conduit transversal et s'étendant sur l'ensemble du déplacement de ce conduit, depuis ladite position distale de non blocage jusqu'à ladite position proximale de blocage.

Ces lumières permettent le passage du ou des fils de part et d'autre de la pièce intérieure lorsque cette pièce intérieure a un diamètre extérieur ajusté au diamètre intérieur de la cavité que forme la pièce extérieure.

De préférence, la pièce intérieure présente deux saillies d'encliquetage aptes à venir s'encliqueter derrière les bords de la pièce extérieure délimitant les extrémités distales desdites lumières.

Un montage et une rétention facile de la pièce intérieure dans la pièce extérieure est ainsi obtenu, par encliquetage.

De préférence, ladite pièce intérieure présente une portion distale pointue, faisant saillie au-delà de la pièce extérieure, du côté distal, lorsque ladite pièce intérieure est dans ladite position distale de non blocage.

Cette extrémité distale pointue favorise l'insertion et la progression de l'ancre dans le trou de la paroi osseuse.

L'invention, telle qu'exposée ci-dessus, sera bien comprise, et d'autres caractéristiques et avantages de celle-ci apparaîtront, en référence au dessin schématique annexé, qui représente, à titre d'exemple non limitatif, une forme de réalisation préférée de l'ancre concernée.
La figure 1 en est une vue en perspective, avec une pièce intérieure qu'elle comprend dans une position sortie par rapport à une pièce extérieure qu'elle comprend également ;
la figure 2 en est une vue en coupe longitudinale dans un plan médian passant par l'axe d'un conduit transversal que forme ladite pièce intérieure, cette pièce intérieure étant sortie par rapport à ladite pièce extérieure ;
la figure 3 en est une vue similaire à la figure 2, selon un plan de coupe perpendiculaire à celui selon la figure 2 ;
la figure 4 en est une vue similaire à la figure 1, après mise en place de fils de suture et avec la pièce intérieure placée dans la pièce extérieure, cette pièce intérieure étant dans une position distale de non blocage des fils de suture ;
la figure 5 en est une vue similaire à la figure 4, selon un plan de coupe identique à celui selon la figure 2 ; cette figure 5 montre également une portion d'extrémité d'une barre et une portion d'une tige que comprend un instrument servant à la mise en place de l'ancre dans une paroi osseuse ; la portion d'extrémité de la barre est vue partiellement en coupe longitudinale et partiellement en extérieur ;
la figure 6 est une vue de l'ancre similaire à la figure 4, selon un plan de coupe identique à celui selon la figure 3 ;
la figure 7 en est une vue avec ladite pièce intérieure dans une position proximale de blocage des fils de suture, selon un plan de coupe identique à celui selon la figure 2 ; et
la figure 8 en est une similaire à la figure 7, selon un plan de coupe identique à celui selon la figure 3.

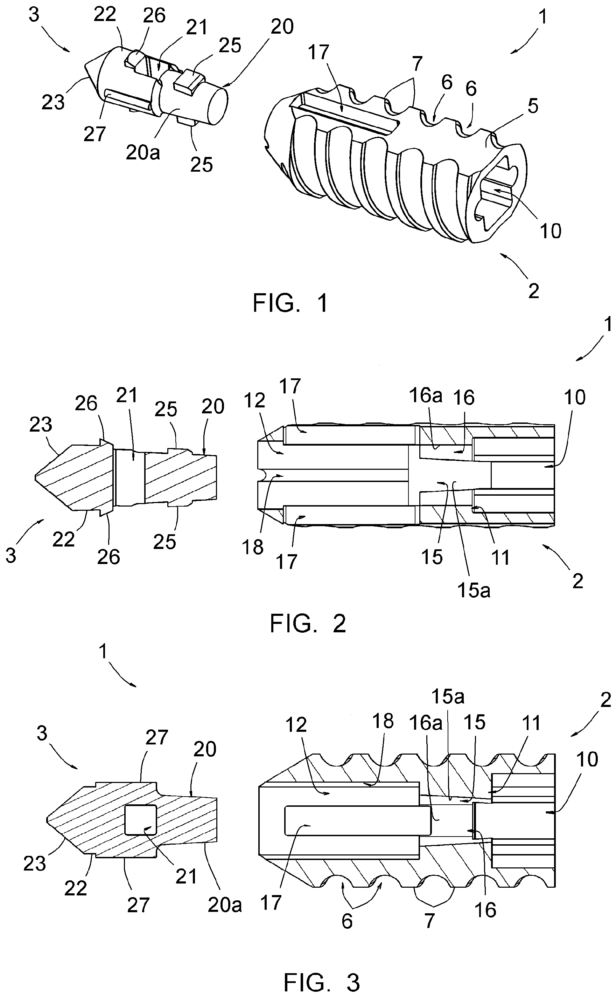

Les figures 1 à 3 représentent une ancre 1 à usage médical, destinée à être insérée dans une paroi osseuse et à fixer ainsi un ou plusieurs fils de suture 100 à cet os. Cette ancre 1 est particulièrement destinée à permettre de réinsérer sur un humérus un ou plusieurs des tendons de la coiffe des rotateurs de l'articulation de l'épaule.

L'ancre 1 comprend une pièce extérieure 2 et une pièce intérieure 3 destinées à être assemblées l'une à l'autre.

La pièce extérieure 2 est destinée à être fixée à l'os. À part une portion distale conique, elle a une section transversale en forme d'anneau aplati, faisant qu'elle présente deux méplats 5 sur deux côtés opposés et deux faces courbes entre ces méplats 5. Dans ces faces courbes, sont aménagées des séries de cannelures profondes 6, délimitant des séries de nervures 7 entre elles. La pièce 2 est apte, dans une première position angulaire d'insertion dans un trou aménagé dans la paroi osseuse, à être engagée dans ce trou, puis à être pivotée en force dans ce trou, jusqu'à une autre position angulaire, décalée de l'ordre de quatre-vingt-dix degrés par rapport à ladite première position angulaire, de façon à coincer l'ancre 1 dans le trou.

La pièce extérieure 2 forme une cavité longitudinale interne la traversant de part en part, délimitant une cavité proximale 10, une portion intermédiaire 11 et une cavité distale 12.

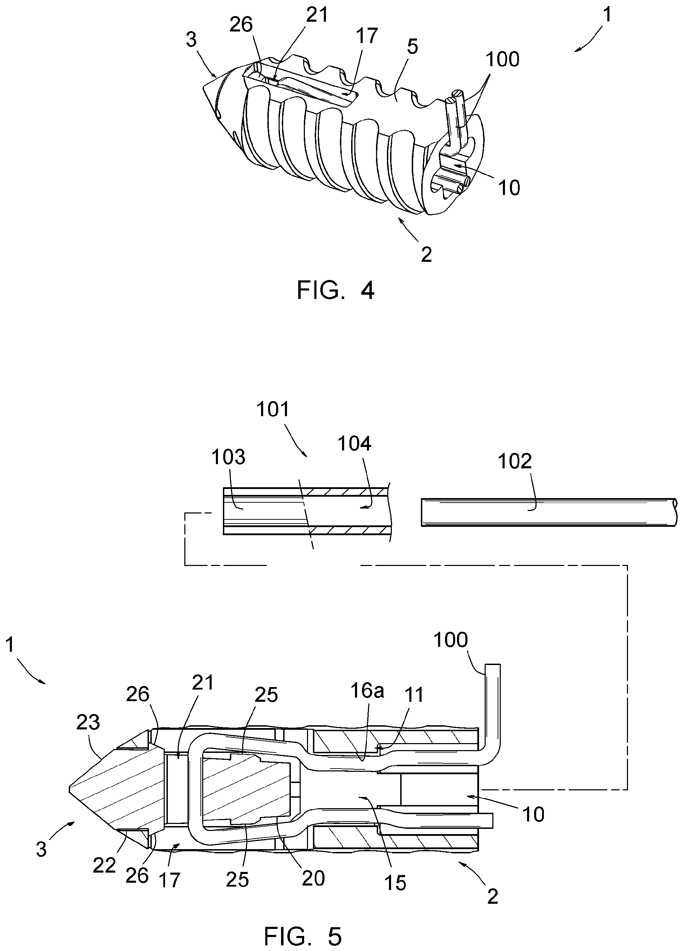

La cavité proximale 10 forme, le long des méplats 5, deux logements latéraux destinés à recevoir les fils de suture, comme visible sur la figure 4, et une cavité de montage centrale incluant deux rainures latérales, destinée à recevoir de façon ajustée l'extrémité distale de la barre 101, de section correspondante.

La portion intermédiaire 11 délimite une cavité conique femelle 15 ayant une pente faible, de l'ordre de 5 à 10 degrés, cette cavité 15 étant apte à recevoir en elle, avec coincement, une portion tronconique mâle 20 que comprend la pièce intérieure 3, décrite plus loin. Elle forme deux conduits 16 diamétralement opposés, situés en dessous des méplats 5 et situés dans le prolongement des deux logements latéraux précités que forme la cavité 10. Ces conduits 16 communiquent avec ces logements latéraux et avec la cavité distale 12, comme visible sur la figure 2 et permettent ainsi le passage au travers d'eux des brins des fils de suture 100, comme visible sur la figure 5.

Il apparaît sur la figure 2 que les parois 16a délimitant les fonds de ces conduits 16 sont parallèles à l'axe longitudinal de la pièce extérieure 2.

La cavité distale 12 communique avec l'extérieur de la pièce 2 par deux lumières 17, s'étendant au travers des méplats 5, et forme deux rainures 18 diamétralement opposées, s'étendant dans un plan diamétral perpendiculaire au plan dans lequel s'étendent les lumières 17.

La pièce intérieure 3 est destinée à être insérée et retenue dans la cavité distale 12 et dans la cavité femelle 15 de la pièce extérieure 2. Du côté proximal au côté distal, elle présente la portion tronconique mâle 20 précitée, une portion traversée par un conduit transversal 21, une portion d'encliquetage 22 et une portion distale 23.

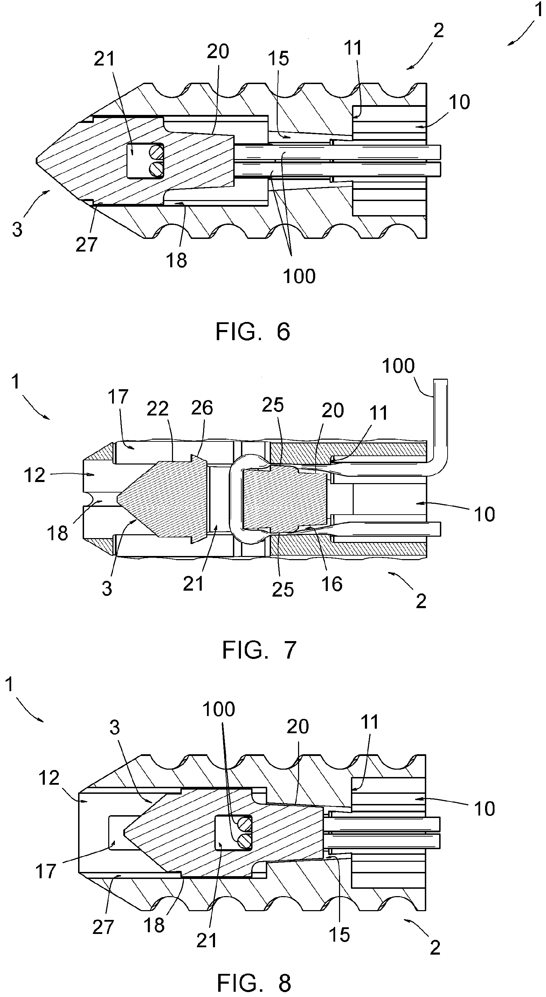

La portion tronconique mâle 20 a une pente correspondant à celle de ladite cavité femelle 15, de façon à être apte à être reçue avec coincement dans cette cavité. Elle forme une face proximale perpendiculaire à son axe longitudinal, contre laquelle la tige 102 coulissant dans la barre 101 est apte à venir en appui, comme décrit plus loin. Elle présente également deux bossages 25 diamétralement opposés, aménagés sur un diamètre de la portion 20 parallèle à l'axe du conduit transversal 21 ; ces bossages 25 sont destinés à s'engager dans lesdits conduits 16 lors du passage de la pièce intérieure 3 de la position distale de non blocage montrée sur les figures 5 et 6 à la position proximale de blocage montrée sur les figures 7 et 8, venant ainsi coincer les brins des fils de suture 100 entre eux et les parois 16a formant les fonds des conduits 16.

Le conduit transversal 21 traverse la pièce intérieure 3 de part en part et présente, dans l'exemple représenté une section transversale carrée. Il est destiné à être traversé par les fils de suture 100, comme visible sur les figures 5 à 8.

La portion d'encliquetage 22 présente deux saillies d'encliquetage 26 aptes à venir s'encliqueter derrière les bords de la pièce extérieure 2 délimitant les extrémités distales des lumières 17, comme visible sur la figure 5.

La portion distale conique 23 est pointue et sa paroi périphérique vient, dans la position distale de non blocage montrée sur la figure 5, dans le prolongement de la paroi de la portion distale conique de la pièce extérieure 2.

En outre, la pièce intérieure 3 forme, sur sa portion délimitant le conduit 21 et sur sa portion d'encliquetage 22, deux nervures allongées 27 diamétralement opposées, situées dans un plan perpendiculaire au plan dans lequel s'étend l'axe du conduit 21. Ces nervures 27 forment des patins destinés à être reçus dans la glissière que forment les rainures 18, comme visible sur les figures 6 et 8.

L'instrument partiellement représenté sur la figure 5 est conforme à celui décrit par la publication de demande de brevet français N° 16 52026. La barre 101 présente une section transversale carrée et, comme visible sur la figure 5, au niveau de sa portion d'extrémité distale, deux nervures latérales 103. Cette portion distale de la barre 101 est adaptée à être engagée dans la cavité de montage précitée que forme la cavité proximale 10, avec les nervures 103 reçues dans les deux rainures latérales précitées de cette cavité.

La barre 101 est percée longitudinalement d'un alésage 104 dans lequel la tige 102 précitée est engagée de façon coulissante. La tige 102 est mobile dans cet alésage 104 entre une position distale, dans laquelle elle vient en appui contre la face proximale de la pièce intérieure 3 alors que la barre 101 est insérée dans ladite cavité de montage, et une position proximale, dans laquelle elle est reculée vis-à-vis de cette face proximale. Dans sa position distale, la tige 102 maintient la pièce intérieure 3 dans la position distale de non blocage et, dans sa position proximale, ne fait pas obstacle au déplacement de la pièce intérieure 3 vers la position proximale de blocage visible sur les figures 7 et 8.

L'instrument comprend des moyens (non représentés, décrit dans la publication de demande de brevet français N° 16 52026) permettant de bloquer sélectivement la tige 102 vis-à-vis de la barre 101, dans ladite position distale de cette tige. Ces moyens peuvent notamment être sous la forme d'un tiroir porté transversalement par un manche solidaire de la barre 101, ce tiroir étant mobile transversalement à l'axe longitudinal de cette barre 101 ; le tiroir présente une portion de paroi pleine et une portion de paroi percée d'un trou de section supérieure à celui de la tige 102 ; dans une position de coulissement, ladite portion de paroi pleine de ce tiroir se trouve à proximité immédiate de l'extrémité proximale de la tige 102 et bloque ainsi tout recul de cette tige, et dans une autre position de coulissement de ce tiroir, le trou que forme ce tiroir se trouve en regard de la tige 102 et permet le recul de cette tige.

En pratique, les fils de suture 100 sont engagés au travers de la pièce extérieure 2, puis au travers du conduit transversal 21 puis à nouveau au travers de la pièce extérieure 2, et la pièce intérieure 3 est engagée dans la cavité distale 12, avec les nervures 27 dans les rainures 18, jusqu'à encliquetage des saillies 26 au-delà des bords de la pièce 2 délimitant les extrémités distales des lumières 17. L'ancre 1 est alors dans l'état visible sur les figures 4 à 6.

Pour réaliser l'insertion de l'ancre 1 dans une cavité osseuse, la portion d'extrémité libre de la barre 101 est engagée dans ladite cavité de montage et la tige 102 est bloquée dans sa position distale, maintenant ainsi la pièce intérieure 3 dans ladite position distale de non blocage visible sur les figures 5 et 6.

Une fois l'insertion de l'ancre 1 dans l'os réalisée, le recul de la tige 102 est débloqué, ce qui permet le déplacement de la pièce intérieure 3 vers la position proximale de blocage visible sur les figures 7 et 8 lorsqu'une traction est exercée sur les fils 100, en particulier lors de la remise en place des ligaments sur l'os. Dans cette position, les fils de suture 100 sont pincés entre les fonds 16a des conduits 16 et les faces radialement externes des bossages 25, ces fonds et faces constituant des surfaces de coincement respectives des fils 100.

Simultanément, les parties de la portion tronconique mâle 20 non en regard des conduits 16 viennent en engagement dans les parties de la cavité 15 situées en dehors des conduits 16, jusqu'à coincement, assurant une immobilisation de la pièce intérieure 3 par rapport à la pièce extérieure 2 dans cette position proximale.

L'invention fournit une ancre à usage médical présentant des avantages déterminants par rapport aux ancres homologues de la technique antérieure, notamment ceux de :
- préserver les brins du ou des fils de suture 100 des détériorations résultant de la mise en place de l'ancre, du fait que ces fils sont placés à l'intérieur de l'ancre 1 ;
- comprendre extérieurement une structure permettant le parfait ancrage de l'ancre 1 dans un os, notamment par coincement résultant d'un pivotement sur un quart de tour ;
- conserver une mise en place relativement facile du ou des fils de suture en elle, du fait de sa structure en deux pièces 2, 3 assemblables ;
- avoir un assemblage facile de ces deux pièces ;
- permettre d'exercer une pression sur une portion relativement importante du ou des fils de suture 100, assurant un parfait coincement de ce ou ces fils, sans risque de cisaillement.

L'ancre 1 a été décrite ci-dessus en référence à une forme de réalisation préférée ; il va de soi que cette description n'est en rien limitative.

## Revendications

1. Ancre (1) à usage médical, destinée à être insérée dans une paroi osseuse, comprenant deux pièces (2, 3) destinées à être assemblées l'une à l'autre, à savoir une pièce extérieure (2), destinée à être fixée à l'os, qui forme une cavité longitudinale interne à travers elle, et une pièce intérieure (3), destinée à être insérée et retenue dans ladite cavité de la pièce extérieure (2), qui forme un conduit transversal (21) la traversant de part en part ; au moins un fil de suture (100) est destiné à être engagé dans ladite cavité par le côté proximal de la pièce extérieure (2), à être engagé au travers dudit conduit transversal (21) et à ressortir de ladite pièce extérieure (2), par ledit côté proximal ;
dans laquelle :
- la pièce extérieure (2) forme au moins une première surface de coincement (16a) disposée dans la direction longitudinale de la pièce extérieure (2), et la pièce intérieure (3) forme au moins une deuxième surface de coincement (25) située du côté proximal par rapport au conduit transversal (21), cette deuxième surface de coincement (25) étant disposée dans la direction longitudinale de la pièce intérieure (3) ;
- ledit au moins un fil de suture (100) est destiné à s'étendre le long de ladite première surface de coincement (16a) et le long de ladite deuxième surface de coincement (25) ;
- la pièce intérieure (3) est mobile par rapport à la pièce extérieure (2) entre une position distale de non blocage du fil de suture (100), dans laquelle ladite deuxième surface de coincement (25) est à une distance de ladite première surface de coincement (16a) ne réalisant aucun coincement de ce fil, et une position proximale de blocage du fil de suture (100), dans laquelle ladite deuxième surface de coincement (25) est à une distance de ladite première surface de coincement (16a) réalisant un coincement de ce fil,
ladite pièce extérieure (2) comprenant deux dites premières surfaces de coincement (16a), diamétralement opposées, et ladite pièce intérieure (3) comprenant deux dites deuxièmes surfaces de coincement (25) correspondantes, au moins une desdites première et deuxième surfaces de coincement (16a, 25) étant inclinée par rapport à l'axe longitudinal de l'ancre (1), et **caractérisée en ce que** :
- ladite pièce extérieure (2) présente une cavité conique femelle (15) dans laquelle sont aménagés deux conduits (16) diamétralement opposés,
- la pièce intérieure (3) présente une portion tronconique mâle (20) ayant une pente correspondant à celle de ladite cavité conique femelle (15), ladite portion tronconique mâle (20) comportant deux bossages (25) diamétralement opposés, aménagés sur un diamètre de la portion tronconique mâle (20) parallèle à l'axe du conduit transversal (21), les bossages (25) étant destinés à s'engager dans lesdits conduits (16) lors du passage de la pièce intérieure (3) de la position distale de non blocage à la position proximale de blocage, venant ainsi coincer le fil de suture (100) entre des faces radialement externes des bossages (25) et des fonds (16a) des conduits (16),
- les fonds (16a) et les faces radialement externes des bossages (25) constituent des premières et deuxièmes surfaces de coincement respectives.

2. Ancre (1) selon la revendication 1, **caractérisée en ce que** ladite pièce extérieure (2) forme au moins une première surface d'engagement (15a), distincte de ladite première surface de coincement (16a), et **en ce que** ladite pièce intérieure (3) forme au moins une deuxième surface d'engagement (20a), distincte de ladite deuxième surface de coincement (25), ces surfaces d'engagement (15a, 20a) respectives venant en engagement mutuel dans ladite position proximale de blocage et réalisant une immobilisation de ladite pièce intérieure (3) par rapport à ladite pièce extérieure (2) dans cette position.

3. Ancre (1) selon la revendication 2, **caractérisée en ce que** lesdites première et deuxième surfaces d'engagement (15a, 20a) sont inclinées par rapport à l'axe longitudinal de l'ancre (1) de manière à être aptes à venir, lorsque la pièce intérieure (3) est déplacée dans le sens proximal à l'intérieur de la pièce extérieure (2), dans un état de coincement mutuel qui détermine ladite position proximale de blocage, et **en ce que** lesdites surfaces de coincement (16a, 25) respectives sont aménagées de telle sorte qu'il existe entre elles, dans cette position proximale de blocage, un espace dont l'épaisseur est de l'ordre de 10 à 40% du diamètre d'un fil de suture (100).

4. Ancre (1) selon la revendication 2 ou la revendication 3, **caractérisée en ce que** :
- ladite pièce extérieure (2) et ladite pièce intérieure (3) présentent des moyens respectifs (18, 27) de positionnement angulaire de ladite pièce intérieure (3) dans ladite pièce extérieure (2), plaçant ledit conduit transversal (21) de la pièce intérieure (3) dans une position angulaire déterminée par rapport à la pièce extérieure (2) ;
- les conduits (16) permettent le passage des brins du ou des fils de suture (100) destinés à être reliés à l'ancre (1) ; ces conduits (16) sont aménagés sur un diamètre qui est parallèle à l'axe dudit conduit transversal (21) lorsque ladite pièce intérieure (3) se trouve dans ladite position angulaire déterminée ; les surfaces délimitant ladite cavité conique femelle (15) entre ces conduits (16) forment deux dites premières surfaces d'engagement (15a) ;
les surfaces de ladite portion tronconique mâle (20) situées en dehors de ces bossages (25) forment deux dites deuxièmes surfaces d'engagement (20a), destinées à venir en engagement avec lesdites premières surfaces d'engagement (15a) dans ladite position proximale de blocage.

5. Ancre (1) selon la revendication 4, **caractérisée en ce que** lesdits moyens respectifs de positionnement sont constitués par deux rainures longitudinales (18) diamétralement opposées aménagées dans ladite pièce extérieure (2) et par des nervures (27) correspondantes aménagés sur la pièce intérieure (3), ces nervures (27) formant des patins destinés à être reçus dans la glissière que forment les rainures (18).

6. Ancre (1) selon l'une des revendications 1 à 5, **caractérisée en ce que** la pièce extérieure (2) présente deux lumières longitudinales (17) diamétralement opposées, situées en regard des ouvertures dudit conduit transversal (21) et s'étendant sur l'ensemble du déplacement de ce conduit (21), depuis ladite position distale de non blocage jusqu'à ladite position proximale de blocage.

7. Ancre (1) selon la revendication 6, **caractérisée en ce que** la pièce intérieure (3) présente deux saillies d'encliquetage (26) aptes à venir s'encliqueter derrière les bords de la pièce extérieure (2) délimitant les extrémités distales desdites lumières (17).

8. Ancre (1) selon l'une des revendications 1 à 7, **caractérisée en ce que** ladite pièce intérieure (3) présente une portion distale (23) pointue, faisant saillie au-delà de la pièce extérieure (2), du côté distal, lorsque ladite pièce intérieure (3) est dans ladite position distale de non blocage.

## Patentansprüche

1. Anker (1) für medizinische Zwecke, der dazu vorgesehen ist, in eine Knochenwand eingesetzt zu werden, mit zwei Teilen (2, 3), die dazu vorgesehen sind, miteinander verbunden zu werden, nämlich einem äußeren Teil (2), das dazu vorgesehen ist, am Knochen befestigt zu werden, und das einen inneren Längshohlraum durch sich hindurch bildet, und einem inneren Teil (3), das dazu vorgesehen ist, in den Hohlraum des äußeren Teils (2) eingesetzt und darin gehalten zu werden, und das einen Querkanal (21) bildet, der sich durch dieses hindurch erstreckt; wobei mindestens ein Nahtfaden (100) dazu vorgesehen ist, von der proximalen Seite des äußeren Teils (2) in den Hohlraum eingeführt zu werden, durch den Querkanal (21) eingeführt zu werden und aus dem äußeren Teil (2) von der proximalen Seite wieder auszutreten;
wobei:
- das äußere Teil (2) mindestens eine erste Klemmfläche (16a) bildet, die in der Längsrichtung des äußeren Teils (2) angeordnet ist, und das innere Teil (3) mindestens eine zweite Klemmfläche (25) bildet, die auf der proximalen Seite in Bezug auf den Querkanal (21) angeordnet ist, wobei diese zweite Klemmfläche (25) in der Längsrichtung des inneren Teils (3) angeordnet ist;
- der mindestens eine Nahtfaden (100) dazu vorgesehen ist, sich entlang der ersten Klemmfläche (16a) und entlang der zweiten Klemmfläche (25) zu erstrecken;
- das Innere Teil (3) in Bezug auf das äußere Teil (2) zwischen einer distalen Position, in der der Nahtfaden (100) nicht geklemmt ist und in der die zweite Klemmfläche (25) von der ersten Klemmfläche (16a) beabstandet ist, so dass kein Einklemmen des Nahtfadens erfolgt, und einer proximalen Position, in der der Nahtfaden (100) geklemmt ist und in der die zweite Klemmfläche (25) von der ersten Klemmfläche (16a) beabstandet ist, so dass ein Einklemmen des Nahtfadens erfolgt, beweglich ist,
das äußere Teil (2) zwei diametral gegenüberliegende erste Klemmflächen (16a) aufweist und das Innere Teil (3) zwei entsprechende zweite Klemmflächen (25) aufweist, wobei mindestens eine der ersten und zweiten Klemmflächen (16a, 25) in Bezug auf die Längsachse des Ankers (1) geneigt ist, und **dadurch gekennzeichnet, dass**:
- das äußere Teil (2) einen konischen Innenhohlraum (15) aufweist, in dem zwei diametral gegenüberliegende Kanäle (16) angeordnet sind,
- das Innenteil (3) einen konischen Außenabschnitt (20) mit einer Neigung aufweist, die der des genannten konischen Innenhohlraums (15) entspricht, wobei der genannte konische Außenabschnitt (20) eine Neigung aufweist, die derjenigen des konischen Innenhohlraums (15) entspricht, wobei der konische Außenabschnitt (20) zwei diametral gegenüberliegende Vorsprünge (25) aufweist, die auf einem Durchmesser des konischen Außenabschnitts (20) parallel zur Achse des Querkanals (21) angeordnet sind, wobei die Vorsprünge (25) dazu vorgesehen sind, beim Übergang des inneren Teils (3) aus der distalen Nicht-Klemmposition in die proximale Klemmposition in die Kanäle (16) einzugreifen, wodurch der Nahtfaden (100) zwischen radial äußeren Flächen der Vorsprünge (25) und den Böden (16a) der Kanäle (16) eingeklemmt wird,
- die Böden (16a) und die radial äußeren Flächen der Vorsprünge (25) jeweils eine erste und eine zweite Klemmfläche bilden.

2. Anker (1) nach Anspruch 1, **dadurch gekennzeichnet, dass** das äußere Teil (2) mindestens eine erste Eingriffsfläche (15a) bildet, die von der ersten Klemmfläche (16a) verschieden ist, und dass das innere Teil (3) mindestens eine zweite Eingriffsfläche (20a) bildet, die von der zweiten Klemmfläche (25) verschieden ist, wobei diese jeweiligen Eingriffsflächen (15a, 20a) in der proximalen Klemmposition in gegenseitigen Eingriff kommen und eine Immobilisierung des inneren Teils (3) in Bezug auf das äußere Teil (2) in dieser Position bewirken.

3. Anker (1) nach Anspruch 2, **dadurch gekennzeichnet, dass** die erste und zweite Eingriffsfläche (15a, 20a) in Bezug auf die Längsachse des Ankers (1) geneigt sind, sodass sie, wenn das innere Teil (3) in proximaler Richtung innerhalb des äußeren Teils (2) verschoben wird, in einen gegenseitigen Klemmzustand gebracht werden können, der die proximale Klemmposition bestimmt, und dass die jeweiligen Klemmflächen (16a, 25) so ausgebildet sind, dass zwischen ihnen in dieser proximalen Klemmposition ein Spalt vorhanden ist, dessen Dicke in der Größenordnung von 10 bis 40 % des Durchmessers eines Nahtfadens (100) liegt.

4. Anker (1) nach Anspruch 2 oder Anspruch 3, **dadurch gekennzeichnet, dass**:
- das äußere Teil (2) und das innere Teil (3) jeweilige Mittel (18, 27) zur Winkelpositionierung des inneren Teils (3) in dem äußeren Teil (2) aufweisen, die den Querkanal (21) des inneren Teils (3) in eine bestimmte Winkelposition in Bezug auf das äußere Teil (2) bringen;
- die Kanäle (16) den Durchgang der Stränge des Nahtfadens oder der Nahtfäden (100) ermöglichen, die dazu vorgesehen sind, mit dem Anker (1) verbunden zu werden; diese Kanäle (16) auf einem Durchmesser angeordnet sind, der parallel zur Achse des Querkanals (21) verläuft, wenn sich das innere Teil (3) in der bestimmten Winkelposition befindet; die Oberflächen, die den konischen Innenhohlraum (15) zwischen diesen Kanälen (16) begrenzen, zwei sogenannte erste Eingriffsflächen (15a) bilden;
- die Oberflächen des konischen Außenabschnitts (20), die außerhalb der Vorsprünge (25) liegen, zwei sogenannte zweite Eingriffsflächen (20a) bilden, die dazu vorgesehen sind, in der proximalen Klemmposition mit den ersten Eingriffsflächen (15a) in Eingriff zu kommen.

5. Anker (1) nach Anspruch 4, **dadurch gekennzeichnet, dass** die jeweiligen Positionierungsmittel aus zwei diametral gegenüberliegenden Längsnuten (18) im äußeren Teil (2) und entsprechenden Rippen (27) im inneren Teil (3) bestehen, wobei die Rippen (27) Kufen bilden, die dazu vorgesehen sind, in der von den Nuten (18) gebildeten Führungsschiene aufgenommen zu werden.

6. Anker (1) nach einem der Ansprüche 1 bis 5, **dadurch gekennzeichnet, dass** das äußere Teil (2) zwei diametral gegenüberliegende Längsschlitze (17) aufweist, die sich gegenüber den Öffnungen des genannten Querkanals (21) befinden und sich über die gesamte Verschiebung dieses Kanals (21) von der distalen Nicht-Klemmposition bis zur proximalen Klemmposition erstrecken.

7. Anker (1) nach Anspruch 6, **dadurch gekennzeichnet, dass** das innere Teil (3) zwei Rastvorsprünge (26) aufweist, die geeignet sind, hinter den Rändern des äußeren Teils (2), die die distalen Enden der Längsschlitze (17) begrenzen, einzurasten.

8. Anker (1) nach einem der Ansprüche 1 bis 7, **dadurch gekennzeichnet, dass** das innere Teil (3) einen spitzen distalen Abschnitt (23) aufweist, der distal über das äußere Teil (2) hinaus vorsteht, wenn sich das innere Teil (3) in der distalen Nicht-Klemmposition befindet.

## Claims

1. Anchor (1) for medical use, intended to be inserted into a bony wall, comprising two pieces (2, 3) intended to be assembled together, namely an outer piece (2), intended to be fixed to the bone, which forms an internal longitudinal cavity through it, and an inner piece (3) intended to be inserted and retained in said cavity of the outer piece (2), which forms a transverse conduit (21) passing through it from one side to the other; at least one suture thread (100) is intended to be engaged in said cavity from the proximal side of the outer piece (2), to be engaged through said transverse conduit (21) and to emerge from said outer piece (2) through said proximal side; wherein:
- the outer piece (2) forms at least one first wedging surface (16a) arranged in the longitudinal direction of the outer piece (2), and the inner piece (3) forms at least one second wedging surface (25) located on the proximal side with respect to the transverse conduit (21), this second wedging surface (25) being arranged in the longitudinal direction of the inner piece (3);
- said at least one suture thread (100) is intended to extend along said first wedging surface (16a) and along said second wedging surface (25);
- the inner piece (3) is movable relative to the outer piece (2) between a distal position in which the suture thread (100) is not blocked, in which said second wedging surface (25) is at a distance from said first wedging surface (16a) that does not wedge the suture, and a proximal position that blocks the suture thread (100), in which said second wedging surface (25) is at a distance from said first wedging surface (16a) that wedges the suture;
said outer piece (2) comprising two said first wedging surfaces (16a), diametrically opposed, and said inner piece (3) comprising two said corresponding second wedging surfaces (25), at least one of said first and second wedging surfaces (16a, 25) being inclined relative to the longitudinal axis of the anchor (1); and
**characterized in that**:
- said outer piece (2) presents a female conical cavity (15) in which two diametrically opposed conduits (16) are arranged;
- the inner piece (3) presents a male truncated conical portion (20) with a slope corresponding to that of said female conical cavity (15), said male truncated conical portion (20) comprising two diametrically opposed bosses (25) arranged along a diameter of the male truncated cone portion (20) parallel to the axis of the transverse conduit (21), the bosses (25) being designed to engage in said conduits (16) when the inner piece (3) moves from the distal non-locking position to the proximal locking position, thereby wedging the suture thread (100) between the radially outer faces of the bosses (25) and the bottoms (16a) of the conduits (16);
- the bottoms (16a) and the radially outer faces of the bosses (25) constitute respectively first and second wedging surfaces.

2. Anchor (1) according to claim 1, **characterized in that** said outer piece (2) forms at least a first engagement surface (15a), distinct from said first wedging surface (16a), and **in that** said inner piece (3) forms at least a second engagement surface (20a), distinct from said second wedging surface (25), said respective engagement surfaces (15a, 20a) coming into mutual engagement in said proximal locking position and achieving immobilization of said inner piece (3) relative to said outer piece (2) in this position.

3. Anchor (1) according to claim 2, **characterized in that** said first and second engagement surfaces (15a, 20a) are inclined relative to the longitudinal axis of the anchor (1) so as to be capable, when the inner piece (3) is moved proximally inside the outer piece (2), of coming into a mutual wedging state that determines said proximal locking position, and **in that** said respective wedging surfaces (16a, 25) are arranged such that, in this proximal locking position, there exists a space between them having a thickness on the order of 10 to 40% of the diameter of a suture thread (100).

4. Anchor (1) according to claim 2 or claim 3, **characterized in that**:
- said outer piece (2) and said inner piece (3) comprise respective means (18, 27) for angular positioning of said inner piece (3) within said outer piece (2), placing said transverse conduit (21) of the inner piece (3) in a predetermined angular position relative to the outer piece (2);
- the conduits (16) allow passage of the strands of the suture(s) (100) intended to be connected to the anchor (1); said conduits (16) are arranged along a diameter that is parallel to the axis of said transverse conduit (21) when said inner piece (3) is in said predetermined angular position; the surfaces delimiting said female conical cavity (15) between these conduits (16) form two said first engagement surfaces (15a);
- the surfaces of said male truncated conical portion (20) located outside these bosses (25) form two said second engagement surfaces (20a), intended to come into engagement with said first engagement surfaces (15a) in said proximal locking position.

5. Anchor (1) according to claim 4, **characterized in that** said respective positioning means consist of two diametrically opposed longitudinal grooves (18) formed in said outer piece (2) and corresponding ribs (27) formed on the inner piece (3), these ribs (27) forming pads intended to be received in the slide formed by the grooves (18).

6. Anchor (1) according to any one of claims 1 to 5, **characterized in that** the outer piece (2) comprises two diametrically opposed longitudinal slots (17), positioned opposite the openings of said transverse conduit (21) and extending over the entire movement of this conduit (21), from said distal non-locking position to said proximal locking position.

7. Anchor (1) according to claim 6, **characterized in that** the inner piece (3) comprises two latching projections (26) capable of snapping behind the edges of the outer piece (2) defining the distal ends of said slots (17).

8. Anchor (1) according to any one of claims 1 to 7, **characterized in that** said inner piece (3) comprises a distal portion (23) pointedly protruding beyond the outer piece (2) on the distal side, when said inner piece (3) is in said distal non-locking position.
